# EUROPEAN PATENT APPLICATION

(11) **EP 1 077 257 A1**
(43) Date of publication of application: **21.02.2001**
(21) Application number: 00906662.2
(22) Date of filing: 03.03.2000
(51) Int. Cl.: C12N 15/11, C12N 5/14, A01H 5/00

(54) **PHOTOINHIBITORY PROMOTER**

(30) Priority: 12.03.1999 JP 6655199
(71) Applicant: SUNTORY LIMITED, Osaka-shi, Osaka 530-8203 (JP)
(72) Inventor: SASAKI, Yukiko, Nagoya-shi, Aichi 464-0817 (JP); NAGANO, Yukio, Nagoya-shi, Aichi 465-0091 (JP); INABA, Takehito, Showa-ku, Nagoya-shi, Aichi 466-0827 (JP)
(74) Representative: HOFFMANN - EITLE
(86) International application number: JP0001269
(87) International publication number: WO0055313

(57) **Abstract**

The present invention provides a DNA fragment or promoter for expressing a gene of interest light-repressibly or specifically in the dark.

A light-repressible promoter was obtained from the 5' upstream region of a plant gene expressed light-repressibly or specifically in the dark, and the function of said promoter was extensively analyzed to reveal a cis-element sequence and a core sequence involved in light-repressible expression. An expression cassette comprising a DNA fragment carrying each of these sequences upstream of a gene of interest can be constructed and transfected into a plant cell or a plant to provide a plant cell or a plant that expresses the gene of interest light-repressibly or specifically in the dark.

## Description

### FIELD OF THE INVENTION

The present invention relates to a promoter for activating expression of a gene of interest in a plant in the dark or for repressing expression of said gene of interest in the light. The present invention also relates to methods for controlling gene expression in a plant using said light-repressible promoters in response to light and dark. More specifically, the present invention relates to light-repressible promoters of a small G protein gene from pea, and methods for using said promoters. PRIOR ART

The gene expression control mechanism of eukaryotes including plants is understood as follows (Kyozuka: "Molecular Mechanism of Plant Morphogenesis", pp. 107 - 117, Shujunsha, 1994). Each gene of an organism has a genetically strictly defined expression pattern emerging in various stages of the life cycle of the organisms. Organisms are able to maintain their function as a living creature because each gene shows a correct expression pattern. Tissue-specific expression (spatially and temporally regulated expression) is an important mechanism of gene expression. Especially during development, morphogenesis and the growth of individuals, tissue-specific expression of a specific gene (genes) provides basic information for maintaining a pattern and advancing subsequent processes.

Regulation of gene expression occurs at both transcriptional and post-transcriptional levels, and the former is more common and has been more extensively analyzed. In transcriptional regulation of expression, most information determining the expression pattern of a gene is thought to be contained in a promoter region on the 5' side of a transcription region. Promoters are DNA regions, which determine the transcription start site of a gene and directly control the frequency of transcription. The region functions as a promoter merely when RNA polymerase or transcription factors are bound to the region. All genes encoding proteins are transcribed by RNA polymerase II.

Promoters often contain various cis-elements. Cis-elements are regions influencing the transcription activity of a gene on the same DNA molecule containing a transcription region. In promoters of many genes, the following points are known: (1) positive/negative cis-elements exist; (2) a plurality of tissue-specific cis-elements are often involved in specific transcription in a specific tissue (such as seed, leaf, pollen, etc.) to independently or jointly determine the transcription pattern and the amount of transcripts; (3) a plurality of cis-elements exist as modular units on a promoter of each gene to determine a tissue-specific transcription pattern unique to the gene as a result of total coordination.

Since the establishment of gene recombination techniques in plants, many transformed plants have been commercialized. Promoters generally used for controlling expression of foreign genes introduced into these transformed plants include constitutive expression promoters such as the cauliflower mosaic virus 35S promoter, nopaline synthase promoter, etc. However, constitutive expression of foreign genes may adversely affect (i.e. impose a penalty) on the transformed plants themselves. Although the gene expression in eukaryotes including plants is controlled by tissue, time, outer environments and other factors, a useful foreign gene can be expressed in a suitable tissue at a suitable time in a suitable environment by transfecting a plant with an expression cassette containing a suitable gene transcription regulatory region (promoter) inserted upstream of the foreign gene. To accomplish this, a promoter for expressing a gene of interest in a suitable tissue at a suitable time in a suitable environment is required. It is industrially useful to tissue-specifically express foreign genes. If regulation could be employed which prevents expression of foreign genes in edible parts of plants, for example, safety risks would be reduced, and public acceptance enhanced.

Light-mediated regulation of gene expression is very important for morphogenesis and growth of plants so that they carry genes whose transcription is activated or repressed by light. If a promoter region of such a light-controllable gene is obtained and linked upstream of a foreign gene to prepare a light-controllable expression cassette, and said light-controllable expression cassette is introduced into a plant, the expression of the foreign gene in said plant can be controlled by light. Thus, adverse influences of constitutive expression on transformed plants themselves can be avoided by controlling the expression of foreign genes by light.

After germination of plants, stems rapidly elongate in soil and, upon appearance above soil and exposure to light, this elongation ceases and leaves develop to start photosynthesis. These changes are mostly controlled by photoreceptor-mediated regulation of gene expression. The small G protein gene pra2 from pea (Nagano et al., 1993, Plant Cell Physiol. 34:447-455) is controlled by a photoreceptor, phytochrome (Yoshida et al., 1993, Proc. Natl. Acad. Sci. USA 90:6636-6640). The pra2 gene is thought to be involved in the elongation of stems during germination in the dark because it is expressed at the epicotyl elongation site of pea and the expression is repressed by light.

Many genes whose expression is activated by light or up-regulated by phytochromes have been reported. Examples are the pea ribulose 1,5-disphosphate carboxylase small subunit rbcS (Sasaki et al., 1983, Eur. J. Biochem. 133:617-620), light-harvesting chlorophyll proteins Lhcb from Lemna gibba (Kehoe et al., 1994, Plant Cell 6:1123-1134), etc. Some of them have been subjected to extensive analyses about transcription factors that are trans-factors involved in transcription regulation (Terzaghi and Cashmore, 1995, Annu. Rev. Plant Physiol. Plant Mol. Biol. 46, 445-474). These promoters containing cis-elemens have also been used to up-regulate expression of foreign genes in plants by light or activate transcription/expression by light. For example, it is reported that light-induced synthesis of cytokinin occurred in tobacco plants in which a gene for cytokinin synthesis ipt linked downstream of the 3A promoter of said rbcS was introduced (Thomas J. C. et al., 1995, Plant. Mol. Biol. 27:225-235).

However, a limited number of reports are directed to genes that are down-regulated by phytochromes or whose transcription/expression is repressed by phytochromes and few are directed to cis-elements involved in their regulation. The promoter of the phytochrome A gene phyA has been well analyzed and a cis-element repressed by phytochromes, RE1 sequence, has been identified (Bruce et al., 1991. EMBO J. 10:3015-3024), but any transcription factor binding to it has not been identified yet. Other genes known to be repressed by light include the soybean β tubulin gene tubB1 (Tonoike et al., 1994, Plant J. 5:343-351), asparagine synthase AS1 (Nagai et al., 1997, Plant J. 12:1021-1034) and one of homeobox genes of Arabidopsis Athb2 (Carabelli et al., 1996, Proc. Natl. Acad. Sci. USA 93:3530-3535), buttheir promoters have not been analyzed in detail, with a few exceptions.

### SUMMARY OF THE INVENTION

The present invention provides a promoter that represses expression of a gene in the light but activates expression of the gene in the dark as well as a cis-element sequence necessary for repressing promoter-induced expression of a gene in the light but activating promoter-induced expression of the gene in the dark.

The present invention also provides a method for repressing expression of a foreign gene by light or activating expression of the foreign gene in the dark using said promoter and/or cis-element.

The present invention also provides an expression cassette for expressing a gene of interest specifically in the dark using said light-repressible promoter and/or cis-element, an expression vector for producing a plant carrying said expression cassette, and a transformed plant obtained by transforming a plant with said expression vector, preferably by introducing a gene of interest into its genome to express said gene of interest specifically in the dark.

### BRIEF DESCRIPTION OF THE DRAWINGS

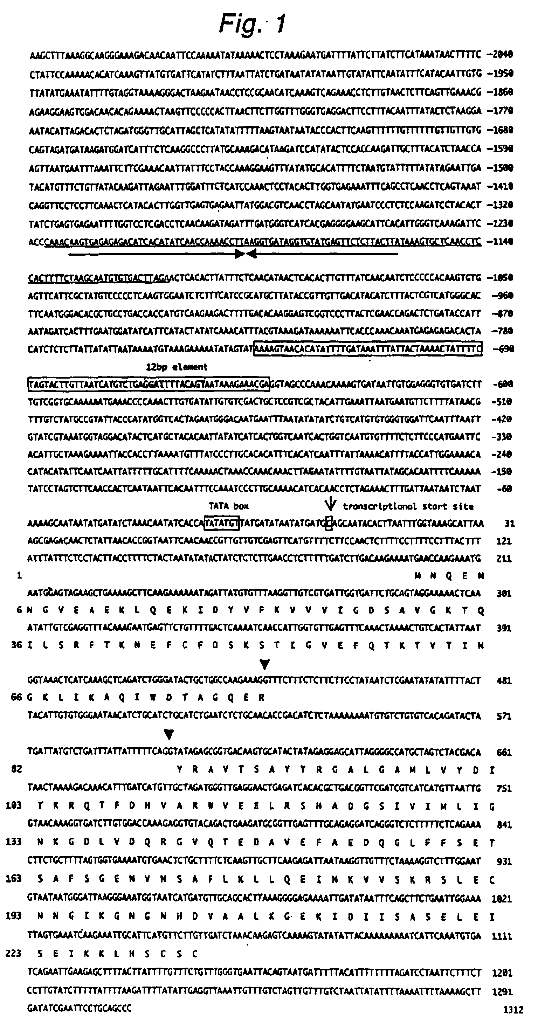

FIG. 1 shows the nucleotide sequence and the amino acid sequence of the pra2 genomic gene referring to the nucleotide sequence number on the right with the transcription start point being 0 (indicated by ↓) and the amino acid sequence number on the left. The arrow heads indicate the exon-intron boundary and the 113-bp inverted homologous sequence is underlined with the inverted repeat sequence being indicated by opposite arrows. The 93-bp cis-element and the TATA box are boxed and the 12-bp core sequence is shaded.

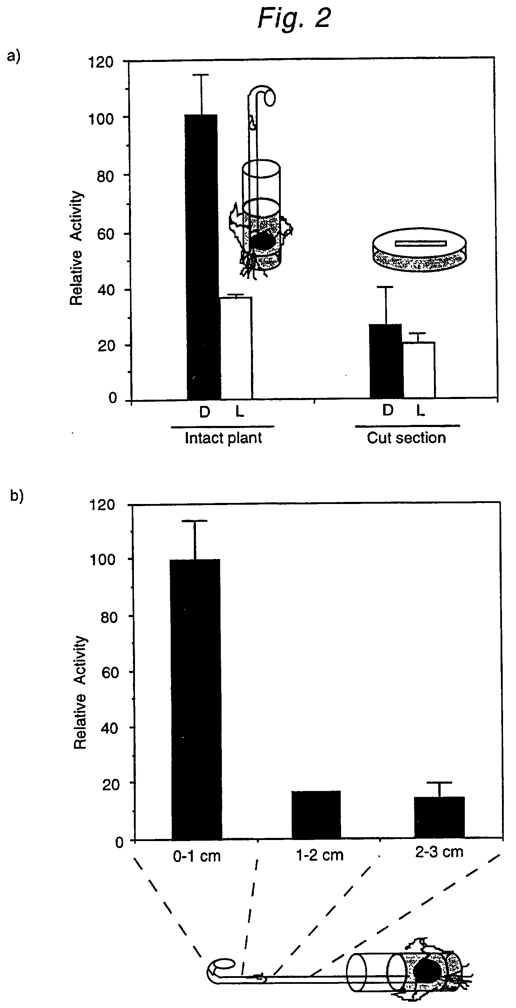

FIG. 2 shows that the pra2 promoter-induced expression of a reporter gene is repressed by light, in which D and L represent the expression levels of the reporter gene (luciferase) after 12 hours in a dark condition and a light condition, respectively. Panel a) shows expression levels of the reporter gene introduced by bombardment of gold particles into the growing part of etiolated stems (left) or a section of the growing part (right), and panel b) shows expression levels at various sites of etiolated stems.

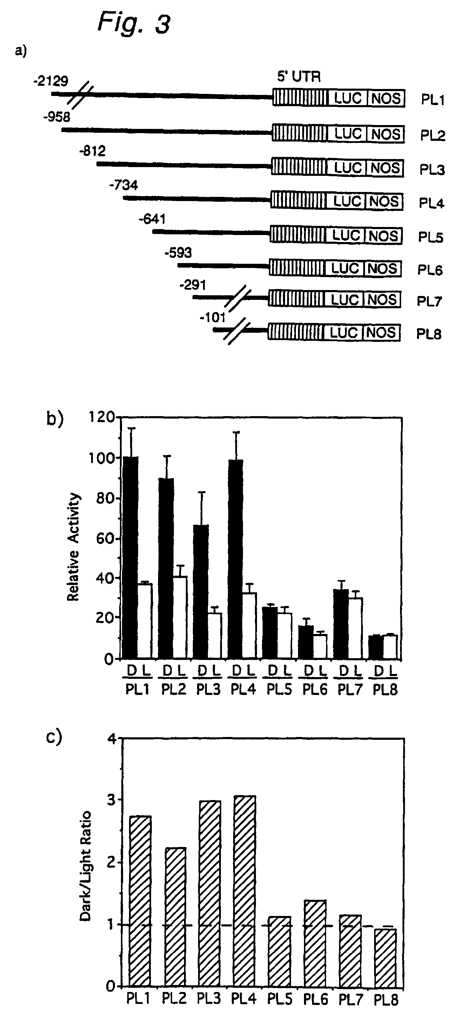

FIG. 3 shows the analytical results of pra2 promoter deletion mutants. Panel a) shows the structures of deletion clones of the pra2 promoter, in which 5'UTR represents the 5' upstream region of the pra2 gene mRNA, LUC represents the luciferase gene and NOS represents the terminator of the nopaline synthase gene. Panel b) shows expression levels of the reporter gene 12 hours after bombardment of deletion clones having the structures shown in panel a) into etiolated stems of pea, in which D and L represent a dark condition and a light condition, respectively. Panel c) shows the ratio of expression levels of the reporter gene in a dark condition and a light condition shown in panel b).

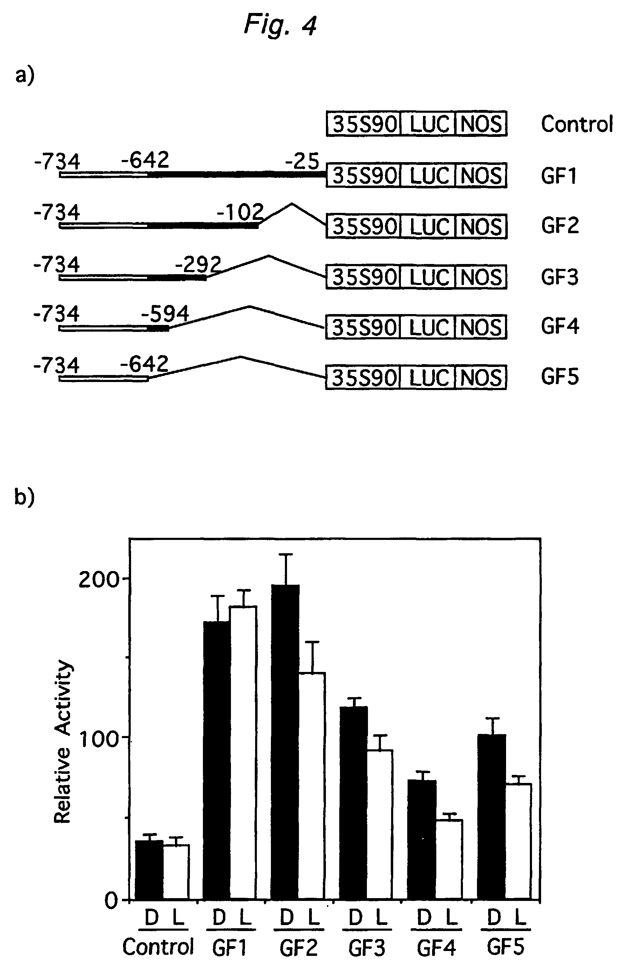

FIG. 4 shows promoter activity in combination with the cauliflower mosaic virus 35S promoter. Panel a) shows the structures of deletion clones of the pra2 promoter, in which the 93-bp cis-element is represented by blank bars and other promoter sites are represented by solid bars. 35S90 represents the cauliflower mosaic virus 35S promoter, LUC represents the luciferase gene and NOS represents the terminator of the nopaline synthase gene. Panel b) shows expression levels of the reporter gene 12 hours after bombardment of deletion clones having the structures shown in panel a) into etiolated stems of pea, in which D and L represent a dark condition and a light condition, respectively.

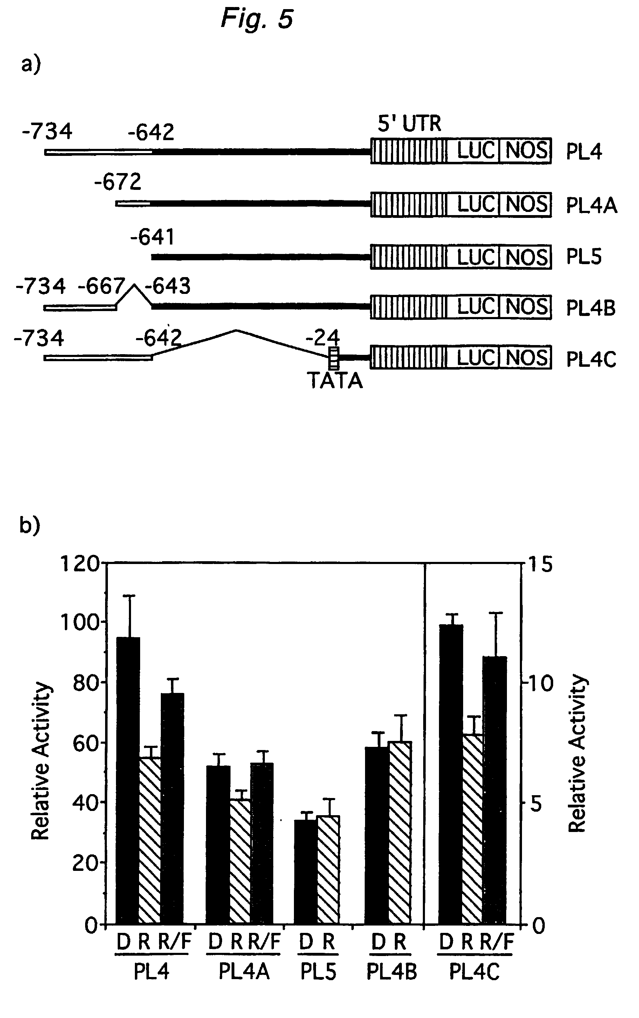

FIG. 5 shows the analytical results of light-repressible cis-elements. Panel a) shows the structures of deletion clones of the pra2 promoter, in which the 93-bp cis-element is represented by blank bars and other promoter sites are represented by solid bars. 5'UTR represents the 5' upstream region of the pra2 gene mRNA, LUC represents the luciferase gene and NOS represents the terminator of the nopaline synthase gene. Panel b) shows expression levels of the reporter gene 12 hours after bombardment of deletion clones having the structures shown in panel a) into etiolated stems of pea, in which D represents a dark condition, R represents a dark condition after red light irradiation for 2 minutes, and R/F represents a dark condition after red light irradiation for 5 minutes followed by near-infrared irradiation for 2 minutes.

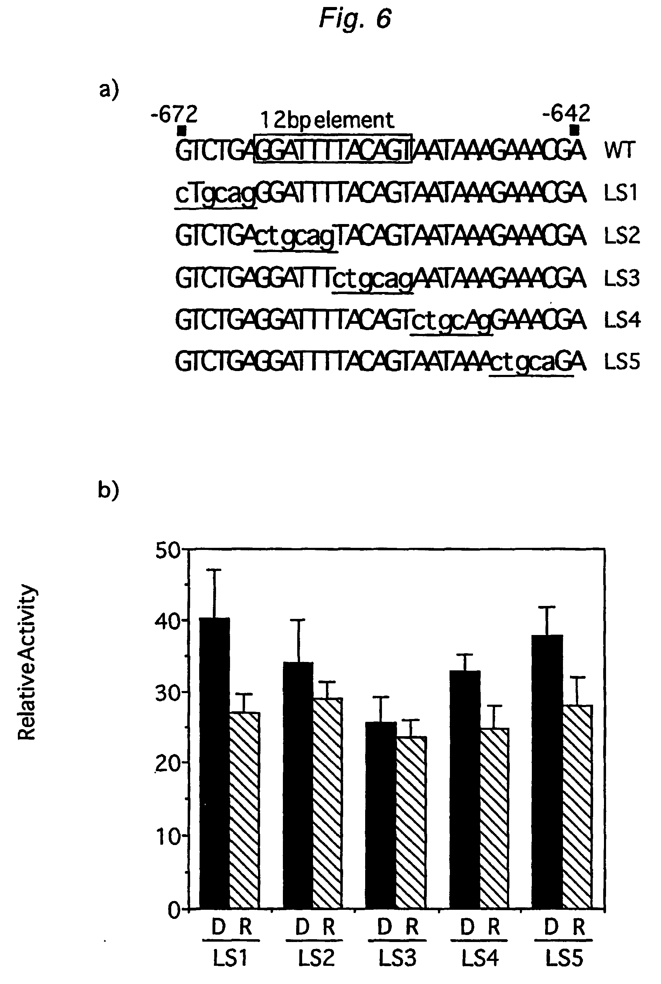

FIG. 6 shows the results of linker scanning analysis of the core sequence. Panel a) shows the nucleotide sequences of the wild type and mutants near the core sequence in the structure of PL4A shown in FIG. 5, with base changes from the wild type being lowercased. Panel b) shows expression levels of the reporter gene 12 hours after bombardment of deletion clones having the structures shown in panel a) into etiolated stems of pea, In which D represents a dark condition and R represents a dark condition for 12 hours after red light irradiation for 2 minutes

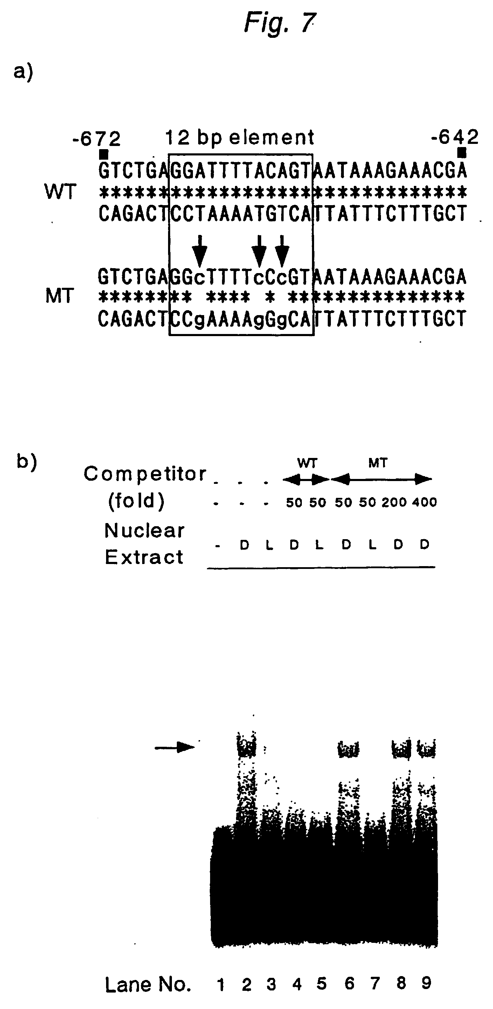

FIG. 7 shows the results of a gel shift assay. Panel a) shows the sequences of synthetic DNAs used in the experiment, in which WT and MT represent the sequences of the wild-type and a mutant, respectively. Panel b) shows the results of the gel shift assay, in which D and L represent extracts prepared from pea epicotyls grown in the dark or illuminated for 6 hours, respectively. The arrow Indicates the electrophoretic position of synthetic DNA-protein complexes.

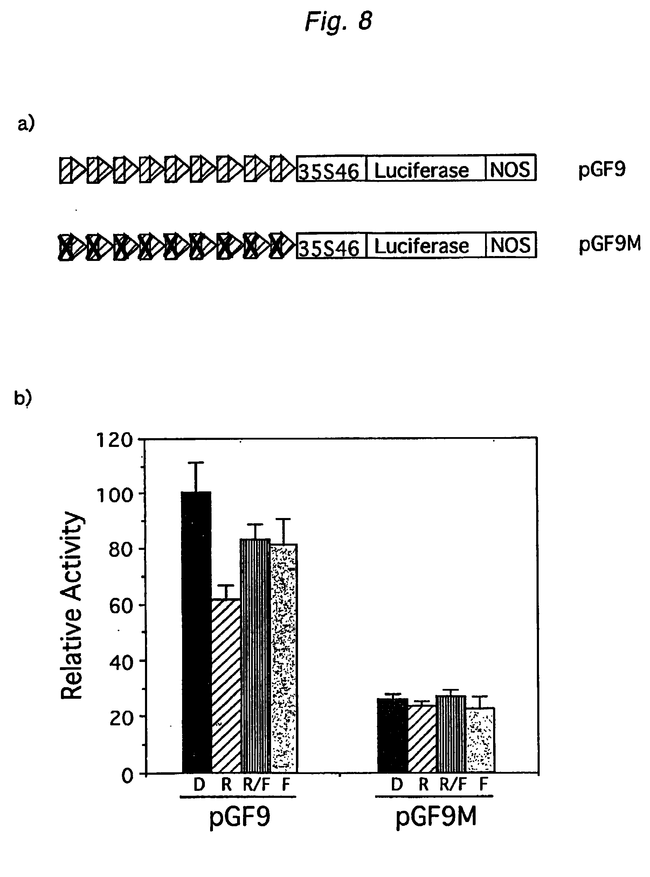

FIG. 8 shows light responsiveness of the 12-bp cis-element. Panel a) shows the structure of pGF9 containing 9 copies of the 12-bp sequence linked upstream of a minimal promoter (CaMV 35S46) and the structure of pGF9M containing 9 copies of the mutant 12-bp cis-element linked upstream of the minimal promoter. Panel b) shows expression levels of the reporter gene 12 hours after bombardment of pGF9 or pGF9M into etiolated stems of pea, In which D represents a dark condition, R represents a dark condition after red light irradiation for 2 minutes, R/F represents a dark condition after red light irradiation for 5 minutes followed by near-infrared irradiation for 2 minutes, and F represents a dark condition after near-infrared irradiation for 5 minutes.

### DETAILED DESCRIPTION OF THE INVENTION

As a result of careful studies of plant-derived genes expressed specifically in the dark, the inventors found that the 5' upstream region of the small G protein gene from pea pra2 has a function of activating expression of the pra2 gene in the dark, i.e. it has a light-repressible promoter function. Extensive analysis of said light-repressible promoter revealed that a 93-bp nucleotide sequence in said promoter is a light-repressible cis-element and that a 12-bp core sequence present in said cis-element is a sequence essential for light-repressible expression. The inventors also found that expression of a gene of interest is activated in the dark and repressed in the light by inserting a promoter containing said core sequence or cis-element (which may be said light-repressible promoter or a combination with another constitutive promoter) upstream of the gene of interest. The inventors also found that a 12-bp cis-element consisting of the 12-bp core sequence alone confers light repressibility on the expression of a gene placed downstream of said element, and thus accomplished the present invention.

Accordingly, the present invention provides a light-repressible promoter and/or cis-element sequence that represses expression of a gene in the light but activates expression of the gene in the dark.

More specifically, the present invention provides a promoter and/or cis-element sequence containing the nucleotide sequence of SEQ ID NO: 1 or 2 as a cis-element, whereby expression of a gene placed downstream of said sequence is repressed by light or activated in the dark, as well as a DNA fragment having said promoter or cis-element function. The 12-bp sequence of SEQ ID NO: 1, the 93-bp sequence of SEQ ID NO: 2 containing said 12-bp sequence and modified sequences obtained by deletion, substitution and/or addition of one or more nucleotides in a part of said 93-bp sequence other than said 12-bp sequence disclosed herein are cis-elements or cis-factors necessary for light-mediated repression of expression. Thus, all the sequences containing the DNA fragments or promoters as defined in claims 1 to 6 are included in the scope of the present invention. A 12-bp cis-element consisting of the 12-bp core sequence alone is sufficient to confer light repressibility on the expression of a gene placed downstream of said element. The term light as used herein means visible light and near-infrared rays, but not infrared rays or ultraviolet rays.

Light-repressible promoters of the present invention can regulate expression of various genes placed downstream of said promoters according to the presence or absence of light. However, light-repressible promoters of the present invention may be combined with promoters originally associated with a gene whose expression is to be regulated by light or promoters of other origins. Such promoters are preferably constitutive expression promoters. The term constitutive expression as used herein means permanent expression independent of surrounding conditions such as the presence or absence of light. Therefore, the present invention also provides a promoter that combines said light-repressible promoter or its cis-element with a constitutive expression promoter to light-repressibly regulates expression of a gene of interest. Various constitutive expression promoters are suitable for the purpose of the present invention. For example, promoters used for gene expression in plant cells include the cauliflower mosaic virus 35S promoter and nopaline synthase promoter. However, constitutive expression promoters may not be necessarily limited to these examples. Constitutive expression promoters for expressing a gene in a host other than plant cells such as a host having phytochromes such as green algae can also be combined with said light-repressible promoter or its cis-element to direct light-regulated production of a gene product by the host. A part of constitutive expression promoters such as a part of the cauliflower mosaic virus 35S promoter, i.e. a minimal promoter up to -72 (CaMV 35S46) can also be used for this purpose.

The present invention also provides a light-repressible expression cassette carrying a gene of interest placed downstream of said light-repressible promoter or cis-element to express said gene of interest light-repressibly or inducibly in the dark. Such a cassette may further contain other sequences useful for expression of the gene of interest such as ribosome-binding site, enhancer or terminator, and may further contain promoters originally associated with the gene of interest or foreign promoters downstream of said light-repressible promoter or cis-element. The inventors also found that a 12-bp cis-element consisting of the 12-bp core sequence alone is sufficient to confer light repressibility on the expression of a gene placed downstream of said element (see Example 9). The expression cassette can be integrated into an appropriate expression vector for use in cell transformation. The expression cassette or expression vector may contain selectable markers for facilitating selection of cells transformed with a gene of interest such as antibiotics resistance genes. Especially suitable cells for transformation are plant cells.

The present invention also provides a plant cell transformed with said light-repressible expression cassette and a recombinant plant obtained by culturing and regenerating said transformed plant cells. Methods for transforming a plant cell with an expression cassette to stably integrate a gene of interest into the chromosome of the plant cell using a particle gun, Agrobacterium, etc. are well known. Methods for growing the transformed plant cell in a medium for plants to form a callus and further growing said callus into a whole plant are well known. Plants that can be transformed and regenerated into whole plants include, but are not limited to, rose, chrysanthemum, carnation, snapdragon, cyclamen, orchid, lisianthus, freesia, gerbera, gladiolus, gypsophila, kalanchoe, lily, pelargonium, geranium, petunia, torenia, tulip, rice, barley, wheat, rapeseed, potato, tomato, poplar, banana, eucalyptus, sweet potato, soybean, alfalfa, lupine, maize, cauliflower. Methods for establishing a stable transformant as a cultivar by crossing the regenerated plants are also well known.

Expression cassettes of the present invention can be used to transform a commercial crop to achieve quality improvement or prevent deterioration of the field crop during storage in the dark, for example. They also can be used for, but are not limited to, the following purposes.
1. A gene encoding an enzyme degrading ethylene or its precursor can be linked to a cis-element or promoter of the present invention and introduced as an expression cassette into a vegetable produced in a plant factory to inhibit ethylene production only during post-harvest storage in the dark, thus preventing overgrowth or overmaturity of the vegetable.
2. A protease degrading a specific protein allergen can be expressed in a crop such as rice or wheat to remove the allergen in the crop.
3. Thioredoxin can be expressed to recombine S-S bonds of proteins in a crop, thus removing allergenicity.
4. A cellulase gene can be expressed to raise the nutritive value of a crop, thus providing a highly digestible food material.
5. An amylase gene can be expressed in a vegetable or fruit to degrade starches in the vegetable or fruit, thus providing a sweet taste.
6. Respiration in mitochondria can be inhibited to prevent quality deterioration of vegetables.
7. An insecticidal protein can be expressed in field crops to protect post-harvest crops from insect damage.
8. The luciferase gene can be used to generate a plant that is photogenic in the dark.
9. A plant that is aromatic at night can be generated.

The present invention is explained more in detail below.

The phenomenon that expression of a gene is repressed by light or activated in the dark is regulated by a promoter placed upstream of the gene. The inventors hypothesized that a light-repressible promoter of the invention can be achieved if an upstream region of a gene whose expression is repressed by light is obtained and the function of this upstream region is analyzed in detail to identify the sequence of a cis-element involved in light-repressible gene expression.

Thus, the inventors screened a pea genomic gene library using cDNA of the small G protein gene (pra2) from pea as a probe to obtain the genomic gene of pra2 (see Example 1). This pra2 genomic gene was found to contain a 2325-bp 5' upstream region. The inventors further analyzed the transcription start point by the primer extension method to find that pra2 mRNA contains a 196-bp 5' upstream region and that this pra2 genomic gene contains a 2129-bp transcription regulatory region (promoter region) (see Example 1, Fig. 1).

The inventors transfected a DNA fragment carrying a reporter gene linked downstream of this 2325-bp 5' upstream region into a pea plant using a particle gun and analyzed the expression of the reporter gene and found that this 5' upstream region light-repressibly regulates gene expression, i.e. it has a light-repressible promoter function (see Example 2). Then, the inventors prepared various deletion clones of this 5' upstream region and analyzed light-repressible expression in plants by the method described above to find that the 93-bp sequence of SEQ ID NO: 2 is a cis-element involved in light-repressible expression (see Examples 3 and 4). The inventors also found that promoters combining said cis-element with other promoters also light-repressibly regulate gene expression (see Examples 5 and 6). The inventors also used a linker scanning assay and a gel shift assay to find that the 12-bp core sequence of SEQ ID NO: 1 present in said cis-element is a region essential for light-repressible expression and that said 12-bp cis-element alone is sufficient to confer light repressibility on the expression of a gene placed downstream of said element (see Examples 7 and 8).

The following examples further illustrate the present invention. Unless otherwise specified, molecular biology techniques were based on Molecular Cloning (Sambrook et al., 1989).

### EXAMPLES

### Example 1: Isolation of the pra2 genomic gene and determination of the transcription start point

A pea genomic gene library (Stratagene) was screened by plaque hybridization according to the method of Nagano et al. (Nagano et al., 1993, Plant Cell Physiol. 34:447-455) using the pra2 cDNA (Nagano et al., 1993, Plant Cell Physiol. 34:447-455) as a probe to isolate a pra2 genomic gene clone. The nucleotide sequence of the pra2 genomic gene is shown in Fig. 1. The genomic gene contained two exons and one intron. The amino acid sequence deduced from the genomic gene differed at one position from the amino acid sequence deduced from the cDNA (Nagano et al., 1993, Plant Cell Physiol. 34; 447-455). Namely, the 206th amino acid in the genomic gene was alanine instead of glycine in cDNA. This may be attributed to the difference of the variety of pea used for isolation of the genomic gene and the cDNA gene.

Then, the transcription start point was determined by primer extension (Nagano et al., 1991, Curr. Genet. 20: 431-436). The primer used was a chemically synthesized primer having the nucleotide sequence:
5'-ACGGTTGTTGAATTACCGGTGTTAATAGAG-3'.
The synthetic primer labeled with ³²P-ATP was hybridized to 1.1 µg of polyA⁺ RNA and transcribed reversely using Superscript II (Gibco BRL). Electrophoresis of the product and analysis of its nucleotide sequence revealed that the genomic gene has a 196-bp 5' upstream region (Fig. 1). The reduced TATA box was shown to be located 24 bp upstream of the translation initiation point.

### Example 2: Establishment of a transient assay system

Seeds of pea (Pisum sativum cv. Alaska, Snow Brand Seed) were sown in a pot having a diameter of 14 mm in the dark and grown for 5-6 days in the dark. This plant was horizontally placed in a particle gun (bombardment apparatus, Model GIE-III, Tanaka). This apparatus has been previously described by Takeuchi et al. (Takeuchi et al., 1992, Plant Mol. Biol. 18:835-839). The growing part (1.0 cm from the apex) of etiolated stems was bombarded with gold particles having a diameter of 1.5-3 µm. The gold particles were coated with a plasmid DNA containing the luciferase gene under control of the pra2 promoter (a 2325-bp 5' upstream region consisting of the 196-bp 5' upstream region of mRNA and a 2129-bp region upstream of the former) or the β-glucuronidase (GUS) gene under control of the cauliflower mosaic virus 35S promoter. The GUS gene was cotransfected as an internal standard to normalize the difference in gene transfer efficiency. Five micrograms of each plasmid was mixed with 2 mg of gold particles and suspended in 200 µl of ethanol. Four microliters of the suspension was used for one bombardment. All the procedures were performed in the dark.

After bombardment, the plant was placed under a dark or light (white light at 70 µmole/m²/sec) condition at 25°C for 12 hours. The transfected stems were minced in liquid nitrogen and the resulting powder was suspended in 300 µl 100 mM potassium phosphate (pH 7.8), 1 mM dithiothreitol, 1% Triton X-100, 1mM EDTA. After centrifugation at 15,000 x g at 4°C for 5 minutes, the supernatant was frozen at -80°C and stored until luciferase activity was measured using a PicaGene luciferase assay kit (Wako Industries) according to the method of Miller et al. (Miller et al., 1992, Plant Mol. Biol. Reptr. 10:324-337). Luciferase luminescence was measured with AUTO LUMAT (Berthold). GUS activity was measured by the method using 4-methyl umbelliferyl β-D-glucuronide (Wako Industries) as a substrate (Jefferson et al., 1987, EMBO J. 6:3901-3907), and the concentration of the produced 4-methyl-umbelliferone was measured with Fluoroskan II (Labosystems). Luciferase activity was calibrated on the basis of the GUS activity cotransfected as an internal standard.

Analysis of the activity of the reporter luciferase in the plant showed a clear difference in the activity of pea plants grown in a pot in the presence or absence of light (Fig. 2a). Namely, luciferase expression was about 3-fold higher in plants grown in the dark than plants grown in the light after transfection. Transfection of the same plasmid into different sites of stems showed that luciferase activity was the highest at the stem elongation site (Fig. 2b). These results showed that the 5' upstream region of the pra2 gene represses expression of the reporter gene in a light condition, i.e. it represses a specific expression at the stem elongation site.

### Example 3: Construction of clones lacking the 5' upstream region

Various deletion clones were constructed according to the following methods to determine the cis-acting region involved in light-mediated repression of the 5' upstream region (2129 bp) of the pra2 gene.

[Method 1] The upstream region of the pra2 gene was amplified with a series of upstream region primers containing a HindIII recognition sequence at the 5' end and a primer containing the NcoI recognition sequence corresponding to the start codon ATG of the pra2 gene (NcoI primer: 5'-GGTCCATGGTCTTGTCAAGATC-3'). Deletion clones for linker scanning were constructed with primers containing a change of 6 nucleotides corresponding to the PsI recognition sequence in the upstream region primers (LS constructs). The amplified fragments were subcloned into the EcoRV site of pZEr0-2.1 (Invitrogen) and digested with HindIII and NcoI. HindIII-NcoI fragments were separated by electrophoresis to recover DNA fragments of interest using a DNA extraction kit (Pharmacia). The plasmid pBI221-LUC containing a luciferase gene (described in "Experimental Protocols for Observing Plant Cells", pp. 199-200, Shujunsha) was digested with HindIII and NcoI, and the DNA fragments was linked to the above recovered DNA fragments. The nucleotide sequences of the subcloned DNA fragments were determined to be identical with the sequences of corresponding domains in the 5' upstream region of the pra2 gene. This method 1 was used to construct the following clones using amplification primers shown in parentheses:
PL1 (5'-GGGAAGCTTTAAAGGCAAGGG-3' and NcoI primer), PL3 (5'-ACGTAAAGCTTAAAAATTCACCC-3' and NcoI primer), PL4 (5'-AAATAAAGCTTAAAAGTAACACATA-3' and NcoI primer), PL4B (5'-AAATAAAGCTTAAAAGTAACACATA-3' and 5'-GTACTGCAGTCAGACATGATTAACAAG-3'), PL5 (5'-AAAGAAGCTTGGTAGCCCAAACAA-3' and NcoI primer), LS1 (5'-AAGCTTctgcagGGATTTTACAGTAATAAA-3' and NcoI primer), LS2 (5'-AAGCTTGTCTGActgcagTACAGTAATAAAGAAAC-3' and NcoI primer), LS3 (5'-AAGCTTGTCTGAGGATTTctgcagAATAAAGAAACGAGGTAG-3' and NcoI primer), LS4

(5'-
AAGCTTGTCTGAGGATTTTACAGTctgcagGAAACGAGGTAGCCCAAA-3' and
NcoI primer), LS5 (5'-
AAGCTTGTCTGAGGATTTTACAGTAATAAActgcagAGGTAGCCCAAACAAG-3' and
NcoI primer).

[Method 2] The following clones were constructed by inverse PCR using PL1 as a template and LA-Taq polymerase (Takara) along with amplification primers shown in parentheses: PL2 (5'-TCAATGGGACACGCTGCCTGACCACCATGT-3' and pUC19 primer: 5'-GGCGTAATCATGGTCATAGCTGTTTCCTGTG-3'), PL6 (5'-TGTCGGTGCAAAAAATGAAACCCCAAACTT-3' and pUC19 primer), PL7 (5'-AATGTTTATCCCTTGCACACATTTCACATC-3' and pUC19 primer), PL8 (5'-GCAAAACATCACAACCTCTAGAAAC-3' and pUC19 primer), PL4C (5'-GTTTGGCTGCAGTCGTTTCTTTATTACTGTAAAATCCTC-3' and 5'-CAATACTGCAGTATATGTTATGATATAATATGATGCAGC-3'). The amplified fragments were blunt-ended and self-ligated.

[Method 3] To construct the plasmid Pra2-35S90LUC (GF), the upstream region of pra2 was amplified with Pfu DNA polymerase and two primers containing the EcoRV recognition sequence and the PstI recognition sequence, respectively. The amplified DNA fragments were subcloned into the EcoRV site of pZEr0-2.1 (Invitrogen) and digested with EcoRV and PstI. The recovered EcoRV-PstI fragments were subcloned into the EcoR-PstI site of pBI221-LUC+. Five DNA fragments having different lengths were amplified. Namely, the following clones were constructed with amplification primers shown in parentheses: GF1 (GF primer: 5'-TACTGCAGAAAAGTAACACATATTT-3' and 5'-TGGTGATATTGTTTAGATATCATATTATTGC-3'), GF2 (GF primer and 5'-ATGATATCCAAGGGATTTGGAAAT-3'), GF3 (GF primer and 5'-GTGATATCGGGATAAACATTTTAAGG-3'), GF4 (GF primer and 5'-TTGATATCCCGACAAAGATCACAC-3'), GF5 (GF primer and 5'-GGGATATCTCGTTTCTTTATTACT-3').

[Method 4] To construct PL4B, the upstream region of pra2 was amplified with Pfu DNA polymerase and two primers containing the HindIII recognition sequence and the PstI recognition sequence on the 5' side, respectively. The amplified DNA fragment was digested with HindIII and PstI and then subcloned into the HindIII-PstI site of pZEr0-2.1, and digested again with HindIII and PstI after determination of the sequence. This fragment was subcloned into the HindIII-PstI site of LS5 containing the LUC gene.

### Example 4: Promoter activity analysis of deletion clones

Using meth ods 1 and 2 described in Example 3, eight deletion clones PL1 to PL8 were constructed in which the 5' upstream region of the pra2 gene was successively deleted from the 5' side (Fig. 3a). These deletion clones were transfected into the stem elongation site of pea using a particle gun according to the method described in Example 2 to measure luciferase activity at the stem elongation site under a dark condition and a light condition. Four deletion clones PL1 to PL4 showed comparable luciferase expression levels in the dark and light-mediated repression of luciferase activity (Fig. 3b). However, expression level in the dark was markedly lowered and no more light-mediated repression of expression was observed in PL5 to PL8 (Fib. 3b). This result shows that the cis-element involved in light response is located in the 93-bp region between PL4 and PL5. Luciferase activity ratio between dark and light conditions (D/L ratio) in deletion clones also dramatically changed from PL4 to PL5 (Fig. 3c), indicating that a light-responsive region exists in the 93-bp region, i.e. said 93-bp DNA fragment having the sequence of SEQ ID NO: 2 is the cis-element involved in light-mediated repression of expression. Recovery of luciferase expression level in PL7 suggests that a repressor repressing the expression level is located between -593 and -292 and that an enhancer increasing the expression level is located between -291 and -101.

### Example 5: Effect of combination with another promoter

This 93-bp light-repressible cis-element was tested for the ability to confer light responsiveness on other promoters, i.e. whether or not other promoters function as a light-repressible promoter when combined with the light-repressible cis-element. 5' upstream regions of the pra2 gene deleted at different lengths in the 3' side were fused to the cauliflower mosaic virus 35S (CaMV 35S90) promoter to prepare 5 clones according to the procedure described in method 3 in Example 3 (Fig. 4a). Light responsiveness was observed in GF2 lacking nucleotides -101 to +196, but not in GF1 lacking nucleotides -24 to +196 (Fig. 4b). This seems to be the result of interaction between the cis-element located in the region between -101 and -25 and the as-1 element in the CaMV 35S90 promoter. Light responsiveness was observed in all of the other clones GF3, GF4 and GF5 (GF5 contains the 93-bp light-repressible cis-element alone) (Fig. 4b). These results show that the 93-bp light-repressible cis-element is sufficient to confer light repressibility on a heterologous promoter, CaMV 35S90.

### Example 6: Analysis of phytochrome-responsive elements

Expression of the pra2 gene is regulated by phytochrome, which is a photoreceptor. Thus, an analysis was made to determine whether or not any phytochrome-responsive cis-elements are present in the 93-bp light-repressible cis-element. At first, clones PL4 and PL5 containing or not the 93-bp light-repressible cis-element were tested (Fig. 5a). Dark condition samples were placed in a dark condition for 12 hours post-transfection. Red light samples were placed in a dark condition for 12 hours after red light treatment for 2 minutes post-transfection. Red light/near-infrared treatment samples were placed in a dark condition for 12 hours after red light treatment for 2 minutes followed by infrared treatment for 5 minutes. As a result, PL4 showed a repression of luciferase expression by the red light treatment and a recovery from the repression by the treatment with near-infrared, but PL5 did not show any repression of expression by the red light treatment (Fig. 5b). To examine whether or not the 93-bp light-repressible cis-element alone can confer the phytochrome responsiveness on the pra2 promoter, a clone was constructed in which said cis-element was fused to the 5' upstream region of the TATA box in the upstream region of the pra2 gene (PL4C in Fig. 5a). The result showed that said cis-element alone can confer phytochrome responsiveness though expression level was markedly lowered (Fig. 5b). For further analysis, a clone PL4A containing a deletion between PL4 and PL5 was constructed (Fig. 5a). This clone maintained phytochrome responsiveness, though expression level was lowered as compared with PL4 (Fig. 5b). Another construct lacking internal 24 base pairs from the 31-bp region (-672 to -642) was prepared (PL4B) and examined to show that phytochrome responsiveness disappered (Figs. 5a and 5b). These results show that the phytochrome-responsive cis-element is located in the 31-bp region from -672 to -642 and also suggest that a cis-element influencing expression level is located in the 62-bp region from -734 to -673.

### Example 7: Determination of 12-bp core sequence by linker scanning

To determine the core sequence in the cis-element involved in red light-mediated repression of the expression of a reporter gene, said 31-bp region was analyzed by linker scanning. Five DNA fragments having changes in a 6-bp region at different positions were prepared (Fig. 6a). The dark condition and red light treatment condition were the same as the conditions described in Example 6. As a result, LS2 and LS3 did not show red light responsiveness any more (Fig. 6b). Especially, LS3 showed no light responsiveness, indicating the presence of a core sequence in the region where the linker was inserted. All the clones other than LS3 showed light responsiveness. These results show that a 12-bp core sequence (5'-GGATTTTACAGT-3') is present in the phytochrome-responsive cis-element. This 12-bp core sequence is a novel core sequence in phytochrome-responsive cis-elements because it is not present in light- or phytochrome-responsive cis-elements so far reported.

### Example 8: Detection of a factor binding to the 12-bp core sequence by a gel shift assay

To determine the presence of any nuclear factor specifically binding to the 12-bp core sequence, a gel shift assay was performed on nuclear extracts of pea epicotyls. The nuclear extracts were prepared from pea plants grown in the dark (6 days) and pea plants illuminated for 6 hours before nuclear extraction (illuminated sample). The nuclear extracts were prepared according to the method of Ishiguro et al. (Ishiguro et al., 1992). The stem of 1 cm from the apex was minced and homogenized in 250 ml of a suspension buffer (10 mM PIPES-KOH [pH 7.0], 1M hexylene glycol, 10 mM magnesium chloride, 5 mM β-mercaptoethanol, 1 mM phenylmethylsulfonyl fluoride (PMSF), 8 µM pepstatin A, 2.4 µM leupeptin). After the homogenate was filtered, nuclei were precipitated by centrifugation at 2,700 x g for 15 minutes and suspended in 50 ml of a washing buffer (50 mM Tris-HCl [pH 7.5], 10 mM magnesium chloride, 20% glycerol, 5 mM β-mercaptoethanol) and centrifuged at 5,200 x g for 15 minutes. This cycle was repeated three times. The precipitate was dissolved in 3 ml of a nuclear lysis buffer (15 mM PIPES-KOH [pH 7.51, 1 .25 M potassium chloride. 5 mM magnesium chloride, 2.5 mM dithiothreitol, 1 mM phenylmethylsulfonyl fluoride, 8 µM pepstatin A, 2.4 µM leupeptin). Insoluble components were removed by centrifugation at 5,200 x g for 15 minutes followed by further centrifugation at 100,000 x g for 1 hour. The supernatant was dialyzed and further centrifuged at 12,000 x g for 15 minutes, and the supernatant was recovered and stored at -80°C.

A gel shift assay was performed according to the method of Shimizu et al. (Shimizu et al., 1996, Plant Mol. Biol. 31: 13-22). A synthetic DNA (WT1) having the same sequence as that of the 31-bp region from -672 to -642 was used as a synthetic primer and end-labeled with ³²P-ATP (Fig. 7a).
WT1 5'-GTCTGAGGATTTTACAGTAATAAAGAAACGA-3'
WT2 5'-TCGTTTCTTTATTACTGTAAAATCCTCAGAC-3'

The labeled WT1 was hybridized with a synthetic DNA (WT2). When 8 µg of the nuclear extracts in 20 µl of a binding buffer (20 mM Tris-HCl [pH 8.0], 50 mM potassium chloride, 0.5 mM EDTA, 15 mM magnesium chloride, 10% glycerol, 1 mM dithiothreitol, 2 µg poly[dI-dC]-poly[dI-dC]) was added to this hybrid, a band showing the formation of a DNA-protein complex was detected (Fig. 7b). This band was shown to be clearly weak in illuminated samples. To examine whether this band of a DNA-protein complex is attributed to a protein specifically bound to the 12-bp core sequence, a mutant DNA (hybrid of MT1 and MT2) was prepared by replacing adenine in the 12-bp core sequence by cytosine (Fig. 7a).
MT1 5'-GTCTGAGGCTTTTCCCGTAATAAAGAAACGA-3'
MT2 5'-TCGTTTCTTTATTACGGGAAAAGCCTCAGAC-3'

The band almost disappeared with the addition of a 50-fold excess of an unlabeled DNA (hybrid of WT1 and WT2), but the strength of the band indicating the formation of the DNA-protein complex remained almost unchanged even when a 50-fold, 200-fold or 400-fold excess of the competitor (hybrid of MT1 and MT2) was added (Fig. 7b). These results show that the detected band is a complex of the 12-bp core sequence and a nuclear factor specifically binding thereto.

### Example 9: Light responsiveness of the 12-bp cis-element

Example 5 demonstrated that the 93-bp region containing the 12-bp core sequence located in the 5' upstream region of pra2 is sufficient to confer light responsiveness on CaMV 35S90. Now, an analysis was made to determine whether or not the 12-bp cis-element has the ability to confer light responsiveness on the minimal promoter of CaMV 35S (CaMV 35S46) comprising the -46 bp region. CaMV 35S46 is a promoter containing only a TATA box in which the cis-element as-1 located between -72 and -90 has bee deleted. GUS expression is hardly observed in tobacco having a construct containing GUS gene linked to the promoter comprising only up to -72 region. Therefore, the 12-bp cis-element itself can be considered as a light-responsive promoter if the promoter containing the 12-bp cis-element linked to CaMV 35S46 directs light-responsive GUS expression.

At first, CaMV 35S46 was amplified by PCR under the following conditions. PCR reaction was performed using the pBI221-LUC+ vector as a template along with primer 35S46UP 
(5'-
AAGCTTGGATCCCTCGAGCTGCAGGATATCGCAAGACCCTTCCTCTATATAAGGA-3')
 and primer KZ35SDW (5'-TTCCATGGAAAGCTGCCTAGGAGATCCTCT-3') and the PCR product was subcloned into the pZEr0-2 vector. A plasmid was purified from the resulting clone and then treated with the restriction endonucleases HindIII and NcoI to recover the fragment of Interest CaMV 35S46. CaMV 35S46 was inserted into the pBI221-ULC vector digested with HindIIi and NcoI to give a vector 35S46-LUC. However, this vector contained a single nucleotide change as compared with the 35S promoter of the initial pBI221 vector because the HindIII site near the translation initiation point of the luciferase gene in the pBI221-LUC+ plasmid was removed by using the KZ35SDW primer. The nucleotide sequence of the promoter region amplified by PCR was confirmed by sequencing.

An oligonucleotide WT3 
(5'-
TGAGGATTTTACAGTAATTGAGGATTTTACAGTAATTGAGGATTTTACAGTAAT-3')
 having three 18-bp sequences including 3 base pairs added at each end of the 12-bp cis-element was synthesized and phosphorylated at the 5' end and then ligated as a single strand. Then, WT4 
(5'-
ATTACTGTAAAATCCTCAATTACTGTAAAATCCTCAATTACTGTAAAATCTCA-3')
 complementary to WT3 was phosphorylated at the 5'-end and then annealed to said WT3 which had been ligated as a single strand, and the annealed product was inserted into the EcoRV site of pZEr0-2 (Invitrogen) to give a plasmid containing 9 copies of the 18-bp sequence.

To remove the sequence derived from the pZEr0-2 vector, PCR was performed using said plamid containing 9 copies of the 18-bp sequence as a template along with primer 18X9RMDW (5'-GCGATATCCTGGATCCTGAGGATTTT-3') and primer 18X9RMUP (5'-AGCGGCCGCCAGTGTGGATATCATTACTGT-3') having a BamHI site and an EcoRV site, respectively. The amplified fragment was digested with BamHI and EcoRV and inserted into the BamHI-EcoRV site of the 35S46-LUC vector to give pGF9 shown in Fig. 9a. The sequence of the region amplified by PCR was determined by sequencing. Then, a plasmid pGF9M in which three adenines in the 12-bp cis-element of pGF9 are replaced by cytosines was constructed in the same manner as described above by using primer MT3

(5'-TGAGGCTTTTCCCGTAATTGAGGCTTTTCCCGTAATTGAGGCTTTTCCCGTAAT-
3') and primer MT4 (5'-
ATTACGGGAAAAGCCTCAATTACGGGAAAAGCCTCAATTACGGGAAAAGCCTCA-3').

The plasmid pGF9 was transfected into pea epicotyls using a particle gun by the method described in Example 2. After transfection of the plasmid, the plant was illuminated under various conditions and incubated in the dark for 12 hours to measure the activity of the reporter enzyme (Fig. 8b). Red light irradiation for 2 minutes induced only about 60% of the activity of the reporter enzyme after incubation in the dark for 12 hours without illumination (100%) to show that expression of the reporter gene was repressed. Red light irradiation for 2 minutes followed by near-infrared irradiation for 5 minutes abolished the red light-induced repressive effect as evidenced by about 80% of the activity of the control incubated in the dark for 12 hours, equally to near-infrared irradiation for 5 minutes. When the plasmid pGF9M was similarly transfected into pea epicotyls to examine the response to light and dark, neither strong expression in the dark nor reversible regulation by red light and red/near-infrared light was observed (Fig. 8b). These results show that the 12-bp cis-element is involved in strong expression in the dark and the regulation of phytochrome-mediated light-responsive expression and that the 12-bp cis-element itself is sufficient to confer light responsiveness on the minimal promoter (CaMV 35S46).

### ADVANTAGES OF THE INVENTION

As apparent from the foregoing description, a light-repressible promoter sequence, a 93-bp light-repressible cis-element sequence present in said promoter and a 12-bp core sequence present in said cis-element are disclosed herein. DNA fragments having these nucleotide sequences can be used to express a gene of interest in a plant cell or a plant light-repressibly or specifically in the dark.

## Claims

1. A DNA fragment containing the sequence of SEQ ID NO: 1 as a core sequence, whereby expression of a gene placed downstream of said DNA fragment is repressed in the presence of light.

2. The DNA fragment of Claim 1 which is a cis-element containing the sequence of SEQ ID NO: 2 or a nucleotide sequence obtained by deletion, substitution and/or addition of one or more bases in a part of the sequence of SEQ ID NO: 2 other than the core sequence of SEQ ID NO: 1, whereby expression of a gene placed downstream of said DNA fragment is repressed in the presence of light.

3. The DNA fragment of Claim 1 comprising the nucleotide sequence of SEQ ID NO: 3.

4. A promoter containing the nucleotide sequence of SEQ ID NO: 1 as a core sequence, whereby expression of a gene placed downstream of said promoter is promoted in the dark but repressed in the presence of light.

5. The promoter of Claim 4 containing the sequence of SEQ ID NO: 2 or a nucleotide sequence obtained by deletion, substitution and/or addition of one or more bases in a part of the sequence of SEQ ID NO: 2 other than the core sequence of SEQ ID NO: 1.

6. The promoter of Claim 4 comprising the nucleotide sequence of SEQ ID NO: 3.

7. The DNA fragment of any one of Claims 1 to 3 having a constitutive expression promoter sequence linked downstream of said DNA fragment.

8. The promoter of any one of Claims 4 to 6 having a constitutive expression promoter linked downstream of said promoter.

9. The DNA fragment of Claim 7 wherein the constitutive expression promoter is cauliflower mosaic virus 35S promoter.

10. The promoter of Claim 8 wherein the constitutive expression promoter is the cauliflower mosaic virus 35S promoter.

11. An expression cassette comprising a DNA fragment carrying a gene linked downstream of the DNA fragment or promoter of any one of Claims 1 to 10, whereby expression of said gene is repressed by light.

12. A plant cell transformed with the expression cassette of Claim 11 or a DNA fragment containing said cassette.

13. A plant transformed with the DNA fragment of Claim 11 or a progeny thereof, and a part of said plant or progeny.
